(19) Europäisches Patentamt
European Patent Office
Office européen des brevets

(11) **EP 4 339 283 A1**

(12) **EUROPEAN PATENT APPLICATION**
published in accordance with Art. 153(4) EPC

(43) Date of publication:
**20.03.2024 Bulletin 2024/12**

(21) Application number: **22807509.9**

(22) Date of filing: **11.05.2022**

(51) International Patent Classification (IPC):
**C12N 11/02** (2006.01)    **C07K 17/14** (2006.01)
**C12N 5/07** (2010.01)

(52) Cooperative Patent Classification (CPC):
**C07K 17/14; C12N 5/06; C12N 11/02**

(86) International application number:
**PCT/JP2022/019966**

(87) International publication number:
**WO 2022/239810 (17.11.2022 Gazette 2022/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.05.2021 JP 2021080636**

(71) Applicant: **Dai Nippon Printing Co., Ltd.
Tokyo 162-8001 (JP)**

(72) Inventors:
• **FUJIEDA Yoshinori
Tokyo 162-8001 (JP)**
• **AYAME Hirohito
Tokyo 162-8001 (JP)**
• **HASE Masahiko
Tokyo 162-8001 (JP)**

(74) Representative: **Beck Greener LLP
Fulwood House
12 Fulwood Place
London WC1V 6HR (GB)**

(54) **DRY MICROCARRIER AND METHOD FOR PRODUCING SAME**

(57)    A method of manufacturing a dry microcarrier for cell culture, including: immersing a gel microparticle of alginic acid containing a divalent or higher-valent cation in a solvent containing a gelatin; and forming a dry microcarrier by drying the gel microparticle of alginic acid immersed in the solvent, in which the dry microcarrier for cell culture includes a gelatin and alginic acid crosslinked with a divalent or higher-valent cation, and contains the gelatin inside thereof, is used.

EP 4 339 283 A1

**Description**

BACKGROUND OF THE INVENTION

Field of the Invention

The present disclosure relates to a dry microcarrier and a method of manufacturing the same.

Background Art

In fields such as pharmaceutical manufactures, gene therapy, regenerative medicine, and immunotherapy, there is a demand for efficient mass culture of cells and tissues. A microcarrier culture method is known as a mass culture technique for cells and the like. In the microcarrier culture method, for example, cells, a culture solution, and microcarriers that serve as adhesion scaffolds for cells are introduced into a culture vessel, and the culture solution is intermittently stirred to float the cells and microcarriers such that floating cells descend and come into contact with microcarriers to adhere the surfaces of the microcarriers, which allows the cells to proliferate (see, for example, Patent Literature 1). Microcarriers can provide very large attachment and growth surface areas to volume ratio, which is advantageous for the mass culture of cells.
Hitherto, methods of manufacturing cell culture articles are known, which include a step of forming a microcarrier from a microcarrier composition comprising a polygalacturonic acid compound or an alginate compound and a step of forming a dry microcarrier (see, for example, Patent Literature 2); however, the component constituting a dry microcarrier has low cell adhesiveness, and the surface of the microcarrier is separately coated with a cell adhesive substance.
In addition, a cell culture carrier, which is characterized by having a coating layer made of collagen on the surface of beads consisting of granular alginate having a diameter of from 1 to 5 mm (see, for example, Patent Literature 3), is known; however, the inside of the cell culture carrier is not disclosed, and it is not known about the feature that the cell culture carrier is dried.

Citation List

Patent Literature

[Patent Literature 1] WO2010/138702
[Patent Literature 2] JP 2018-516578 A
[Patent Literature 3] JP H5-81 A

SUMMARY OF THE INVENTION

Technical Problem

There is a need to obtain a dry microcarrier that has favorable cell adhesiveness and high storage stability.

Solution to Problem

The present inventors found that it is possible to form a dry microcarrier in a dry state with favorable cell adhesiveness and high storage stability, which is manufactured by method of manufacturing a dry microcarrier for cell culture, comprising: a step of immersing a gel microparticle of alginic acid containing a divalent or higher-valent cation in a solvent containing a gelatin; and a step of forming a dry microcarrier by drying the gel microparticle of alginic acid immersed in the solvent, wherein the dry microcarrier comprises a gelatin and alginic acid crosslinked with a divalent or higher-valent cation, and contains the gelatin inside thereof. The present disclosure is based on these findings.
In other words, according to one aspect of the present disclosure,

provided is a method of manufacturing a dry microcarrier for cell culture, comprising: a step of immersing a gel microparticle of alginic acid containing a divalent or higher-valent cation in a solvent containing a gelatin; and
a step of forming a dry microcarrier by drying the gel microparticle of alginic acid immersed in the solvent,
wherein the dry microcarrier for cell culture comprises a gelatin and alginic acid crosslinked with a divalent or higher-valent cation, and contains the gelatin inside thereof.

According to another aspect of the present disclosure, a dry microcarrier for cell culture, which is manufactured by the method described above, is provided.

According to another aspect of the present disclosure, a dry microcarrier for cell culture comprising a gelatin and alginic acid crosslinked with a divalent or higher-valent cation and containing the gelatin inside thereof is provided.

Advantageous Effects of Invention

The dry microcarrier for cell culture manufactured by the method of the present disclosure is advantageous because it has favorable cell adhesiveness and high storage stability.

BRIEF DESCRIPTION OF THE DRAWINGS

[FIG. 1] FIG. 1 shows a phase contrast microscopy image of gel microparticles of alginic acid.

[FIG. 2] FIG. 2 shows a phase contrast microscopy image of dry microcarriers manufactured by the method of the present disclosure.

[FIG. 3] FIG. 3 shows a Confocal Laser Scanning Microscopy image of a dry microcarrier manufactured by the method of the present disclosure near the center in the Z-axis direction.

[FIG. 4] FIG. 4 shows phase contrast images after one week of cell culture using dry microcarriers manufactured by the method of the present disclosure.

[FIG. 5] FIG. 5 shows fluorescence microscopy images of calcein staining of cells after one week of cell culture using dry microcarriers manufactured by the method of the present disclosure.

[FIG. 6] FIG. 6 shows phase contrast microscope images of cells treated with 10 mM EDTA after one week of cell culture using dry microcarriers manufactured by the method of the present disclosure.

[FIG. 7] FIG. 7 shows phase contrast microscope images for the gelatin concentrations after culture (left side) and fluorescence microscope images of cells stained with calcein (right side).

DETAILED DESCRIPTION OF THE INVENTION

<Method of Manufacturing Dry Microcarrier for Cell Culture>

According to the present disclosure, provided is a method of manufacturing a dry microcarrier for cell culture (also herein referred to as "the dry microcarrier of the present disclosure" or simply referred to as "dry microcarrier"), comprising:

immersing a gel microparticle of alginic acid containing a divalent or higher-valent cation in a solvent containing a gelatin; and
forming a dry microcarrier by drying the gel microparticle of alginic acid immersed in the solvent,
wherein the dry microcarrier for cell culture comprises a gelatin and alginic acid crosslinked with a divalent or higher-valent cation, and contains the gelatin inside thereof

(also herein referred to as "the method of the present disclosure").

In the present disclosure, the "bonding" between components constituting a gel microparticle of alginic acid, and the "bonding" between components constituting a dry microcarrier for cell culture include all forms of bonding, including both bonding involving chemical bonding and bonding not involving chemical bonding. The bonding between components constituting a dry microcarrier for cell culture may include one type of bonding or two or more different types of bondings. The bonding between components constituting a gel microparticle of alginic acid, and the bonding between components constituting a dry microcarrier for cell culture described above include, for example, a bonding between alginic acids, a bonding between gelatins, and a bonding between alginic acid and gelatin.

A "crosslink" is a form of "bonding." The expressions "crosslinked," "bonded via a crosslink," and their similar expressions used in the present disclosure can be used interchangeably, and any of them refers to the reversible or irreversible bonding of the same or different molecules with or without the intermediation of another substance. For example, the expression "alginic acid crosslinked with a divalent or higher-valent cation" refers to the reversible or irreversible bonding of a plurality of alginic acid molecules via a divalent or higher-valent cation. In the present disclosure, unless otherwise defined, the expression "chemical crosslinking" and its similar expression each refer to the irreversible bonding of a plurality of molecules via a covalent bond. The expression "physical crosslinking" and its similar expression each refer to the reversible bonding of two or more molecules via a non-covalent bond. Further, the "physical crosslinking" may involve chemical bonding or may not involve chemical bonding. Examples of physical crosslinking involving chemical bonding include bonding of a plurality of crosslinks via chemical bonding (intermolecular force bonding) such as intermolecular electrostatic attraction (ion-ion interaction), hydrogen bonding, dipolar interaction, and van der Waals force. In addition, examples of physical crosslinking not involving chemical bonding include mechanical adhesion and entanglement between molecules.

Gel Microparticle of Alginic Acid

The gel microparticle of alginic acid containing a divalent or higher-valent cation used in the method of the present disclosure can be formed by reacting droplets containing alginic acid with an aqueous solution containing a divalent or higher-valent cation such that alginic acid is crosslinked via the divalent or higher-valent cation. Crosslinking of alginic acid via a divalent or higher-valent cation is reversible crosslinking based on electrostatic attraction between the anion moiety (e.g., carboxylic acid group) of an alginic acid molecule and a divalent or higher-valent cation (physical crosslinking involving chemical bonding).

Since alginic acid is crosslinked via a divalent or higher-valent cation in the microcarrier of the present disclosure, when the microcarrier of the present disclosure is used, alginic acid is decrosslinked using a substance that inhibits crosslinking by competing with a divalent or higher-valent cation contributing to the crosslinking. As a result, the microcarrier can be dissolved. Examples of such a substance that inhibits crosslinking include a chelating agent and a monovalent cation. These substances that inhibit crosslinking may be used singly or in a combination of two types or more.

As the chelating agent, a chelating agent the same as that described for the above-described microcarrier can be used. The type of chelating agent is not particularly limited. Examples thereof include ethylenediaminetetraacetic acid (EDTA), glycol ether diamine tetraacetic acid (EGTA), 1,2-bis(o-aminophenoxy)ethane-N,N,N',N'-tetraacetate (BAPTA), N'-(2-hydroxyethyl)ethylenediamine-N,N,N'-triacetic acid (HEDTA), and nitrilotriacetic acid (NTA), which are often used in common cell culture. It is particularly preferable to use EDTA among the above-described chelating agents. In addition, the concentration of the chelating agent is not particularly limited and can be set if appropriate depending on the type of chelating agent, the type of solvent that dissolves the chelating agent, and the like. The concentration of the chelating agent can be, for example, from 0.5 to 20 mM, from 0.75 to 15 mM, and from 1 to 10 mM.

The type of monovalent cation is not particularly limited, and examples thereof include a sodium ion and a potassium ion. As for a monovalent cation, the monovalent cation may be dissolved in a solvent containing no monovalent cations or in a solvent containing monovalent cations for use. Alternatively, the monovalent cation may be used in the form of a buffer containing monovalent cations (e.g., PBS). The concentration of the monovalent cation is not particularly limited and can be set if appropriate depending on the type of monovalent cation, the type of solvent that dissolves the monovalent cation, and the like. The monovalent cation concentration may be set to, for example, from 10 to 500 mM, from 30 to 300 mM, or from 50 to 200 mM.

The droplets containing alginic acid can be formed by extruding alginic acid into droplets through a thin nozzle into the air or oil. Then, the droplets are mixed with an aqueous solution containing divalent or higher-valent cations. Thus, microparticles are formed in which the droplets are in a gelled state. For example, calcium alginate-crosslinked gel microparticles of alginic acid can be formed by forming droplets of sodium alginate and mixing the droplets with a calcium chloride aqueous solution.

It is possible to confirm whether alginic acid constituting a microparticle of alginic acid is bonded by crosslinking via a divalent or higher-valent cation as described above by a method for confirming that when water is added to a microcarrier in a dry state, a gel-like particle is formed, following which when EDTA is added thereto, the gel-like particle is dissolved.

The microparticle of alginic acid may contain alginic acid bonded by physical crosslinking as well as alginic acid bonded by crosslinking via the above-described divalent or higher-valent cation. Such physical crosslinking for forming alginic acid may or may not involve chemical bonding. Still, physical crosslinking not involving chemical bonding (e.g., adhesion, entanglement, and the like between alginic acid molecules) is preferable. Therefore, in one embodiment, the microparticle of alginic acid contains, as alginic acid, alginic acid molecules bonded via a divalent or higher-valent cation and alginic acid molecules bonded by physical crosslinking.

Even in the case of a gel in which a general polysaccharide is used instead of the above-described alginic acid, it can be dried as appropriate and can be expected to have the effect of increasing cell adhesiveness. In particular, polysaccharides such as galacturonic acid, carrageenan, dulan gum, and pectin, which gel with a divalent or higher-valent cation, can be dissolved if appropriate. Thus, they can be applied to the method of the present disclosure. Although not bound by theory, polysaccharides, like alginic acid, have two or more carboxylic acid groups (carboxyl groups) and can form a crosslinked structure with a divalent or higher-valent cation if appropriate. Therefore, they are thought to be used for forming dry microcarriers.

In addition, in the case of dispersing gel microparticles of alginic acid in a solution during the manufacture of the dry microcarrier of the present disclosure, it is preferable that the solution contains a divalent or higher-valent cation to maintain a crosslinked structure between alginic acid molecules that constitute gel microparticles of alginic acid as appropriate.

Divalent or Higher-valent Cation

The divalent or higher-valent cation contained in gel microparticles of alginic acid is not particularly limited; however, it is preferably at least one selected from the group consisting of a calcium ion, a magnesium ion, a divalent iron ion, a trivalent iron ion, a divalent silver ion, a trivalent silver ion, a copper ion, a zinc ion, a selenium ion, and other trace element ions essential for humans (e.g., manganese ion, chromium ion, and cobalt ion), and preferably a calcium ion.

Gelatin

A gelatin used in the method of the present disclosure is an inexpensive substance that has high cell adhesiveness and is easily dissolved. The gelatin may or may not be crosslinked. Note that when an irreversible crosslink, such as a chemical crosslink, is formed in the gelatin, it tends to prevent microcarriers from being dissolved by a chelating agent or the like after cell culture. Therefore, it is preferable that an irreversible crosslink, such as a chemical crosslink, is not formed in a gelatin used in the method of the present disclosure. In addition, a gelatin used in the method of the present disclosure is preferably a gelatin that meets the gelatin standards, purified gelatin standards, sterility standards, microbial limit standards, purity standards, or viscosity standards of Japanese Pharmacopoeia (JP), more preferably a gelatin that meets the standards for gelatin or purified gelatin of Japanese Pharmacopoeia (JP).

Meanwhile, when the gelatin is cross-linked, its adsorption to gel microparticles of alginic acid increases, which is preferable for cell culture. Therefore, from the above viewpoints, the gelatin is preferably crosslinked. From the viewpoint of dissolubility of the above-described microcarrier, it is particularly preferable that a reversible crosslink, such as a physical crosslink, is formed in the gelatin. The physical crosslink may involve chemical bonding or may not involve chemical bonding. In addition, the gelatin may include a bonding via the above-described chemical crosslinking as well as a bonding via such physical crosslinking. Here, for crosslinking in the gelatin, it is preferable to adjust the crosslinking conditions such that the crosslinking is weak enough to dissolve the microcarriers after culture easily. Conventionally known methods for crosslinking molecules can be used as a method for crosslinking a gelatin. Specific examples include methods involving thermal crosslinking, ultraviolet crosslinking, and radiation crosslinking. In addition to a gelatin, cell-adhesive substances, including scaffold proteins, such as collagen, laminin, elastin, proteoglycan, fibronectin, and vitronectin, peptides and domains thereof with sequences having activities (e.g., RGD peptide and laminin E8), and serum may also be contained. In such a case, it is preferable that a microparticle contains a gelatin in which an irreversible crosslink is not formed such that the dissolution of the microparticle by a chelating agent or the like after culture is not prevented.

The gelatin concentration in a solvent for immersing a gel microparticle of alginic acid is not particularly limited, but the higher the gelatin concentration, the more favorable the swelling and cell adhesion and proliferation, which is preferable. The gelatin concentration in a solvent for immersing a gel microparticle of alginic acid is preferably more than 0.1% by mass, more preferably 0.5% by mass or more, still more preferably 1% by mass or more, even more preferably 2% by mass or more, particularly preferably 4% by mass or more. In a case in which 1% by mass alginic acid is used for a gel microparticle, the gelatin concentration can be increased to 99% by mass at a maximum. However, since the higher the gelatin solution concentration, the higher the gelatin solution viscosity, from the viewpoint of the handleability of the solution, the gelatin concentration in a solvent for immersing a gel microparticle of alginic acid is preferably 20% by mass or less, more preferably 15% by mass or less, still more preferably 10% by mass or less.

Since a gelatin is contained inside a gel microparticle of alginic acid, swelling can take place favorably by adding water again after drying the gel microparticle of alginic acid. This is because the presence of gelatin between alginic acid molecules tends to prevent a new crosslink from being formed between alginic acid molecules during drying. When dry microcarriers are prepared, it is preferable to prepare dry microcarriers with a gelatin in a negatively charged state because in a case in which a gelatin is positively charged, alginic acid is negatively charged, forming a poorly soluble complex, which may be unlikely to be dissolved. For this purpose, it is preferable to use a gelatin having an acidic isoelectric point, such as an alkali-treated gelatin, because the gelatin is negatively charged in a neutral state. In addition, in the case of using a gelatin having an alkaline isoelectric point, such as an acid-treated gelatin, it is preferable to prepare dry microcarriers while bringing the gelatin into a negatively charged state by increasing the hydroxide ion concentration of a solution used during dry microcarrier preparation because the gelatin is positively charged in a neutral state. Here, since the isoelectric point of the acid-treated gelatin is from 7 to 9, the pH is preferably 9 or higher, particularly preferably from 9 to 13. In the case of using a cell adhesive substance other than gelatin, it is also preferable to prepare dry microcarriers with the cell adhesive substance in a negatively charged state. From the viewpoint of cell adhesiveness, it is preferable that the gelatin is partially exposed on the gel microparticle surface.

The method of the present disclosure comprises a step of immersing a gel microparticle of alginic acid containing a divalent or higher-valent cation in a solvent containing a gelatin. The solvent containing a gelatin is not particularly limited; however, it is preferably water.

In addition, the method of the present disclosure comprises a step of forming a dry microcarrier by drying the gel microparticle of alginic acid immersed in a solvent containing a gelatin. Drying microcarriers allows the volume of each microparticle of alginic acid to decrease such that the microparticle is condensed and alginic acid constituting the microparticle of alginic acid is brought into close contact with a cell adhesive substance such as gelatin. It is thus thought that a physical crosslink is formed between the alginic acid and the cell adhesion substance, allowing the cell adhesiveness of microcarriers after drying (i.e., dry microcarriers) to be improved.

In addition, the dry microcarrier of the present disclosure is advantageous in that since it is in a dry stage after moisture removal, it has high storage stability and is easily sterilized. In general, in the case of a microcarrier in a state of containing a relatively large amount of a liquid component (i.e., a swelling state), a chemical reaction between the liquid component and a component constituting the microcarrier (e.g., hydrolysis) takes place, which may result in the decomposition of the microcarrier. Meanwhile, in the case of a microcarrier in a state of containing substantially no liquid component (i.e., a dry state), a chemical reaction between the liquid component and a component constituting the microcarrier can be inhibited, eventually preventing the microcarrier from being decomposed. In addition, in a case in which a microcarrier containing a relatively large amount of a liquid component is contaminated with a microorganism such as a bacterium, the microorganism may proliferate through such a liquid component. The dry microcarrier of the present disclosure is used for cell culture. Therefore, in a case in which a microorganism proliferates in the dry microcarrier, a cell of interest may be contaminated with a large amount of the microorganism (contamination), eventually making it impossible to kill such a microorganism entirely or inhibit the proliferation of the microorganism using a common antibiotic for cell culture. Meanwhile, when the dry microcarrier is in a state of containing substantially no liquid component, the proliferation of the microorganism via the liquid component can be inhibited, eventually making it possible to prevent the contamination with the microorganism in a cell of interest. Thus, it is possible to kill such a microorganism entirely or inhibit the proliferation of the microorganism using a common antibiotic for cell culture.

When immersing a gel microparticle of alginic acid in a solvent containing gelatin and then forming a dry microcarrier, it is preferable to replace, for example, a solvent (preferably water) containing gelatin outside the gel microparticle of alginic acid with a poor solvent for gelatin before the drying step, thereby allowing the poor solvent for gelatin to surround the gel microparticle of alginic acid, which is infiltrated with gelatin, and then performing drying. By doing so, most of the unnecessary gelatin outside the gel microparticle of alginic acid is removed, making it possible to prevent microcarriers from agglomerating after drying and to perform drying while encapsulating gelatin in the microcarriers, which is preferable. Specifically, for example, a gel microparticle of alginic acid is immersed in a gelatin aqueous solution such that gelatin infiltrates inside the gel microparticle of alginic acid. Subsequently, gelatin outside the gel microparticle of alginic acid is condensed with ethanol (poor solvent for gelatin) (nanoparticle formation) and washed to be removed such that the gel microparticle of alginic acid is surrounded by ethanol. Then, drying may be performed.

According to a preferred aspect of the method of the present disclosure, provided is a method of manufacturing a dry microcarrier for cell culture, comprising:

> a step of immersing a gel microparticle of alginic acid containing a divalent or higher-valent cation in a solvent containing a gelatin;
> a step of immersing the gel microparticle of alginic acid immersed in the solvent containing a gelatin in a poor solvent for gelatin; and
> a step of forming a dry microcarrier by drying the gel microparticle of alginic acid immersed in the poor solvent instead of the solvent,
> wherein the dry microcarrier for cell culture comprises a gelatin and alginic acid crosslinked with a divalent or higher-valent cation, and contains the gelatin inside thereof.

When replacing, for example, a solvent (preferably water) containing gelatin outside the gel microparticle of alginic acid with a poor solvent for gelatin before the drying step, it is preferable to carry out an operation in a manner as to minimize the shear stress due to the poor solvent applied to the gel microparticle of alginic acid to prevent the gelatin on the surface of the gel microparticle of alginic acid from being removed. The poor solvent is preferably a volatile solvent such as ethanol or isopropyl alcohol because it is likely to be removed subsequently.

In addition, mixed droplets of alginic acid and a gelatin are added dropwise to a poor solvent for gelatin containing a divalent or higher-valent cation such that a gel microparticle of alginic acid infiltrated with the gelatin can be surrounded by the poor solvent for gelatin. According to this method, replacing, for example, water outside the gel microparticle of alginic acid with a poor solvent for gelatin can be omitted, which is more preferable.

In addition, it is preferable to carry out a step drying a gel microparticle of alginic acid immersed in a solvent containing a gelatin by vacuum drying, freeze drying, thermal drying, or a combination of these drying methods. The drying time of vacuum drying is preferably 30 minutes or more. It is preferable to carry out thermal drying with a drying temperature of preferably from 100 to 200°C, more preferably from 120 to 160°C, for a drying time of from 0.5 to 6 hours. As the drying temperature is higher and the drying time is longer, gelatin-gelatin bonding in a microcarrier can be strengthened due to physical bonding such as mechanical adhesion or adsorption (physical crosslinking) or chemical bonding between two components (chemical crosslinking) caused by a reaction called the Maillard reaction between alginic acid and a gelatin. However, when the microcarrier is used, it becomes unlikely to be dissolved by a chelating agent, a proteolytic enzyme, or the like. Thus, an excessively higher temperature and an excessively longer time are not preferable.

It is preferable that the gelatin is distributed throughout the inside of the dry microcarrier (i.e., dry microcarrier for cell culture) of the present disclosure. The expression "distributed throughout the inside" used herein does not mean a requirement that a gelatin is uniformly distributed inside a dry microcarrier, but refers to a condition that as long as a gelatin is distributed throughout the inside of a dry microcarrier, some deviation in the distribution of the gelatin inside thereof can be tolerated. In addition, the greater the amount of gelatin in the dry microcarrier of the present disclosure, the more preferable it is. It is more preferable that the amount of gelatin is greater than that of alginic acid in the dry microcarrier. In other words, the gelatin content is preferably greater than the alginic acid content in the dry microcarrier of the present disclosure. It is predicted that the gelatin enters between alginic acid molecules to prevent the alginic acid molecules from being newly crosslinked with a divalent or higher-valent cation during drying, thereby facilitating swelling in the form before drying and inhibiting the agglomeration of microcarriers after drying.

The shape of the dry microcarrier of the present disclosure is not particularly limited; however, it is preferably, for example, a shape such as a spherical, ellipsoidal, rod-shaped, sheet-shaped, or polygonal shape. The dry microcarrier of the present disclosure may be sterilized by a specified sterilization method after drying. Examples of the specified sterilization method include dry heat sterilization, gamma ray sterilization, electron beam sterilization, and EOG (ethylene oxide gas) sterilization.

Dry Microcarrier

A gelatin is contained inside the dry microcarrier of the present disclosure. X-ray photoelectron spectroscopy (XPS), carbon, hydrogen and nitrogen elemental analysis, a reduced pressure chemiluminescence method, or a combination of two or more thereof can be used as a method for confirming whether a gelatin is contained inside the dry microcarrier. In other words, whether or not a gelatin is contained inside the dry microcarrier of the present disclosure is confirmed by any one of or a combination of two or three of XPS, carbon, hydrogen and nitrogen elemental analysis, and a reduced pressure chemiluminescence method. It is preferable to perform carbon, hydrogen and nitrogen elemental analysis and a reduced pressure chemiluminescence method separately or to perform a combination of XPS and carbon, hydrogen and nitrogen elemental analysis or a reduced pressure chemiluminescence method. Specific techniques of XPS, carbon, hydrogen and nitrogen elemental analysis, and a reduced pressure chemiluminescence method are explained below and in the Examples described later.

In XPS, dry microcarriers are ground for measurement before and after pulverization ("before pulverization" means "before grinding" and "after pulverization" means "after grinding;" the same applies hereinafter), and confirmation is carried out based on the change in the value of N 1s/C 1s (atomic %/atomic %). For example, as long as the value of $\alpha$ (after pulverization)/a (before pulverization), where N 1s/C 1s (atomic %/atomic %) before pulverization is designated as $\alpha$ (before pulverization) and N 1s/C 1s (atomic %/atomic %) after pulverization is designated as $\alpha$ (after pulverization), is 0.08 or more, a gelatin is contained inside the dry microcarrier of the present disclosure, which is preferable. Further, considering the state after swelling described later, the value of $\alpha$ (after pulverization)/a (before pulverization) is preferably 0.1 or more, more preferably 0.3 or more, still more preferably 0.4 or more, particularly preferably 0.43 or more. When the value of $\alpha$ (after pulverization)/a (before pulverization) is within the above-described range, sufficient swelling of the dry microcarrier is achieved such that, for example, swelling can take place to the extent that the size of the microcarrier after swelling is with a difference of 50% or less of the size of the microcarrier before drying. Further, considering cell adhesiveness and proliferative capacity of the microcarrier after swelling, the value of $\alpha$ (after pulverization)/a (before pulverization) is preferably 0.45 or more, more preferably 0.5 or more, still more preferably 0.6 or more, particularly preferably 0.69 or more. When the value of $\alpha$ (after pulverization)/a (before pulverization) is within the above-described range, sufficient cell adhesion and cell proliferation can be achieved. The pulverized manufactures of the dry microcarrier are a powder obtained by grinding the dry microcarrier, preferably obtained by grinding the dry microcarrier in a mortar until less than half its original size.

In addition, it is also possible to confirm whether a gelatin is contained inside the microcarrier by combining XPS and carbon, hydrogen and nitrogen elemental analysis or the reduced pressure chemiluminescence method described above. In the case of combining XPS with carbon, hydrogen and nitrogen elemental analysis or the reduced pressure chemiluminescence method, the value of $\beta/\alpha$, where N 1s/C 1s (atomic %/atomic %) obtained by XPS is designated as $\alpha$ and the nitrogen content (% by mass) obtained by the carbon, hydrogen and nitrogen elemental analysis or the reduced pressure chemiluminescence method is designated as $\beta$, is 4.96 or more, a gelatin is contained inside the dry microcarrier, which is preferable. Further, considering the state after swelling described later, the value of $\beta/\alpha$ is preferably 10 or more, more preferably 15 or more, still more preferably 20 or more, particularly preferably 27 or more. When the value of $\beta/\alpha$ is within the above-described range, sufficient swelling of the dry microcarrier is achieved such that, for example, swelling can take place to the extent that the size of the microcarrier after swelling is with a difference of 50% or less of the size of the microcarrier before drying. Further, considering cell adhesiveness and proliferative capacity of the microcarrier after swelling, the value of $\beta/\alpha$ is preferably 30 or more, more preferably 35 or more, still more preferably 40 or more, particularly preferably 44 or more. When the value of $\beta/\alpha$ is within the above-described range, sufficient cell adhesion and cell proliferation can be achieved. The nitrogen content (% by mass) $\beta$ described herein is measured when the dry microcarrier is in an unpulverized state (in the state before pulverization).

Further, in the carbon, hydrogen and nitrogen elemental analysis and the reduced pressure chemiluminescence method, the nitrogen content (Y) of dry microcarriers is measured based on the nitrogen composition ratio as the nitrogen content (% by mass), and the amount of gelatin (A) is calculated with the gelatin composition ratio of the dry microcarrier as the gelatin content (% by mass) based on the nitrogen content (Z) in the case of alginic acid alone and the nitrogen content (X) in the case of gelatin alone.

Specifically, calculation is carried out by the following formula: A = (Y-Z)/(X-Z). Since alginic acid contains substantially no nitrogen, the greater the nitrogen content in the dry microcarrier, the greater the amount of gelatin in a substantially proportional manner.

When measurement is difficult by elemental analysis in the case of alginic acid alone or gelatin alone, the nitrogen content (X) in the case of gelatin alone may be set as 16.2% by mass (measured value) and the nitrogen content (Z) in the case of alginic acid alone may be set as 0.031% by mass (measured value).

In addition, the nitrogen content in the dry microcarrier of the present disclosure is preferably 0.43% by mass or more with respect to the total amount of the dry microcarrier. Further, considering the state after swelling, the nitrogen content is preferably 1% by mass or more, more preferably 2% by mass or more, still more preferably 3% by mass or more, particularly preferably 3.9% by mass or more. When the nitrogen content is within the above-described range, sufficient swelling of the dry microcarrier is achieved such that, for example, swelling can take place to the extent that the size of the microcarrier after swelling is with a difference of 50% or less of the size of the microcarrier before drying. Further, considering cell adhesiveness and proliferative capacity of the microcarrier after swelling, the nitrogen content is preferably 4.5% by mass or more, more preferably 5% by mass or more, still more preferably 6% by mass or more, particularly preferably 6.7% by mass or more. When the nitrogen content is within the above-described range, sufficient cell adhesion and cell proliferation can be achieved.

In one embodiment, the nitrogen content and/or gelatin content in the dry microcarrier is measured and/or calculated by performing any one of the carbon, hydrogen and nitrogen elemental analysis and the reduced pressure chemiluminescence method. In this case, when the nitrogen content in a measurement target is more than 0.5% by mass, measurement is carried out by the carbon, hydrogen and nitrogen elemental analysis, while when the nitrogen content in a measurement target is 0.5% by mass or less, measurement is carried out by the reduced pressure chemiluminescence method.

In another embodiment, the values $\alpha$ and $\beta$ in the dry microcarrier are measured by performing XPS and the carbon, hydrogen and nitrogen elemental analysis or the reduced pressure chemiluminescence method, respectively. In this case, when the nitrogen content in a measurement target is more than 0.5% by mass, measurement is carried out by the carbon, hydrogen and nitrogen elemental analysis, while when the nitrogen content in a measurement target is 0.5% by mass or less, measurement is carried out by the reduced pressure chemiluminescence method.

According to a preferred aspect of the method of the present disclosure, provided is a method of manufacturing a dry microcarrier for cell culture, comprising:

a step of immersing a gel microparticle of alginic acid containing a divalent or higher-valent cation in a solvent containing a gelatin; and
a step of forming a dry microcarrier by drying the gel microparticle of alginic acid immersed in the solvent, wherein the dry microcarrier for cell culture consists of a gelatin and alginic acid crosslinked with a divalent or higher-valent cation, and contains the gelatin inside thereof.

The dry microcarrier of the present disclosure can swell if necessary to be used as a microcarrier for cell culture. Specifically, the dry microcarrier of the present disclosure can swell with water before cell culture. Then, cells are seeded such that the cells can adhere and proliferate on the microcarrier surface. Meanwhile, a microcarrier for cell culture obtained from the dry microcarrier of the present disclosure is allowed to react with a chelating agent such that a divalent or higher-valent cation crosslinked with alginic acid can be removed from the microcarrier and the microcarrier can be dissolved, thereby collecting cells alone.

By adding, for example, an aqueous solution to the dry microcarrier of the present disclosure, the dry microcarrier can be hydrated to swell. It is preferable that swelling of the dry microcarrier is achieved to the extent that the dry microcarrier is allowed to swell such that it has a size or shape similar to that of a gel microparticle of alginic acid immersed in a solvent before drying. As the dry microcarrier is allowed to swell such that it has a size or shape similar to that of a gel microparticle of alginic acid immersed in a solvent before drying, transparency improves, making it easier to observe cells. In addition, the agglomeration of microcarriers is unlikely to occur, facilitating cell culture.

The difference between the size (average particle size) of the microcarrier after swelling (microcarrier swelling with water) and the size (average particle size) of the gel microparticle of alginic acid immersed in water before drying is preferably 50% or less, more preferably 25% or less, still more preferably 10% or less. The difference between the size of the microcarrier after swelling (size after swelling) and the size of the gel microparticle of alginic acid immersed with water before drying (size before drying) can be calculated by the following formula.

[Formula 1]

$$(\text{Difference between size after swelling and size before drying}) \, (\%) = \left| (\text{Size before drying} - \text{Size after swelling}) / (\text{Size before drying}) \times 100 \right|$$

The dry microcarrier of the present disclosure is used as a cell culture carrier. Therefore, it is preferable that the size in use (i.e., after swelling) is larger than 10 $\mu$m, which is the approximate size of a cell. In addition, when the size is excessively large for floating culture, the sedimentation speed is fast, and cultivation is difficult. Therefore, the size is preferably 1 mm or less. Accordingly, the size of the dry microcarrier of the present disclosure in use (i.e., after swelling) is not particularly limited; however, it is preferably 1 mm or less, more preferably 0.6 mm or less, still more preferably 0.4 mm or less. Although the dry microcarrier of the present disclosure is in a dry state, it is usually used in a swelling state for cell culture. Thus, the size of the dry microcarrier in use (i.e., after swelling) is greater than the size thereof in a dry state. Therefore, to adjust the size in use in the above-described range, the size of the dry microcarrier of the present disclosure (i.e., before swelling) is preferably more than 4.5 $\mu$m and 520 $\mu$m or less, more preferably 310 $\mu$m or less, still more preferably 210 $\mu$m or less.

It is preferable that the aqueous solution for swelling the dry microcarrier of the present disclosure contains a cell adhesive substance. This is because the cell adhesive substance can be adsorbed to a dry microcarrier manufactured by the method of the present disclosure, thereby improving cell adhesiveness. As a cell adhesive substance contained in the aqueous solution for swelling, for example, gelatin, collagen, laminin, elastin, proteoglycan, fibronectin, and vitronectin, peptides and domains thereof with sequences having activities (e.g., RGD peptide and laminin E8), serum, or the like is preferably used.

According to a preferred aspect of the method of the present disclosure,
provided is a method of manufacturing a microcarrier for cell culture, comprising:

a step of immersing a gel microparticle of alginic acid containing a divalent or higher-valent cation in a solvent containing a gelatin;
a step of forming a dry microcarrier by drying the gel microparticle of alginic acid immersed in the solvent; and
a step of performing swelling of the dry microcarrier with a solvent (preferably water),
wherein the microcarrier for cell culture comprises a gelatin and alginic acid crosslinked with a divalent or higher-valent cation, and contains the gelatin inside thereof.

According to a more preferred aspect of the method of the present disclosure,
provided is a method of manufacturing a microcarrier for cell culture, comprising:

a step of immersing a gel microparticle of alginic acid containing a divalent or higher-valent cation in a solvent containing a gelatin;
a step of immersing the gel microparticle of alginic acid immersed in the solvent containing a gelatin in a poor solvent for gelatin before drying;
a step of forming a dry microcarrier by drying the gel microparticle of alginic acid immersed in the poor solvent instead of the solvent; and
a step of performing swelling of the dry microcarrier with a solvent (preferably water),
wherein the microcarrier for cell culture comprises a gelatin and alginic acid crosslinked with a divalent or higher-valent cation, and contains the gelatin inside thereof.

According to another aspect of the method of the present disclosure,

provided is a method of manufacturing a microcarrier for cell culture, comprising:

a step of immersing a gel microparticle of alginic acid containing a divalent or higher-valent cation in a solvent containing a gelatin, thereby forming a microcarrier,
wherein the microcarrier for cell culture comprises a gelatin and alginic acid crosslinked with a divalent or higher-valent cation, and contains the gelatin inside thereof.

According to another preferred aspect of the method of the present disclosure,
provided is a method of manufacturing a microcarrier for cell culture, comprising:

a step of immersing a gel microparticle of alginic acid containing a divalent or higher-valent cation in a solvent containing a gelatin; and
a step of immersing the gel microparticle of alginic acid immersed in the solvent containing a gelatin in a poor solvent for gelatin, thereby forming a microcarrier,
wherein the microcarrier for cell culture comprises a gelatin and alginic acid crosslinked with a divalent or higher-valent cation, and contains the gelatin inside thereof.

In any of the above-described aspects, the amount or distribution of gelatin in the microcarrier for cell culture can be confirmed by drying the microcarrier for cell culture and performing a technique such as XPS, as explained in the Examples described later.

Cell Culture

A dry microcarrier manufactured by the method of the present disclosure is allowed to swell with the aqueous solution described above, following which cells are seeded, allowing the cells to adhere to the microcarrier surface for cell proliferation. For proliferation, cells may be in a static state or cultured while the microcarrier is floating by stirring the medium or the like. To culture a larger number of cells using the microcarrier, floating culture is preferable.

Adhesive cells are preferably used as cells that are used with the microcarrier such that the cells can be cultured on the microcarrier surface. Examples of such cells include parenchymal cells of the liver such as hepatocytes, Kupffer cells, endothelial cells such as vascular endothelial cells and corneal endothelial cells, fibroblasts, osteoblasts, osteoclasts, periodontal ligament-derived cells, epidermal cells such as epidermal keratinocytes, tracheal epithelial cells, gastrointestinal epithelial cells, cervical epithelial cells, epithelial cells such as corneal epithelial cells, mammary gland cells, pericytes, myoblasts, myotube cells, satellite cells, muscle cells such as smooth muscle cells and cardiomyocytes, kidney cells, pancreatic islets of Langerhans cells, nerve cells such as peripheral nerve cells and optic nerve cells, cartilage cells, and bone cells. These cells may be primary cells directly collected from tissues or organs or may be those that have been subcultured several times. Furthermore, these cells may be any of undifferentiated cells such as embryonic stem cells and iPS cells, somatic stem cells such as mesenchymal stem cells that have differentiation potential, unipotent stem cells such as vascular endothelial progenitor cells that have unipotency, and cells that have completed differentiation. Cells that are used with the microcarrier may further be any of CHO cells that are widely used as cell base materials for biopharmaceutical and viral vector manufactures, 293 cells, 3T3 cells, Vero cells, MRC5 cells, HeLa cells, hybridomas, and the like, and established cell lines with these lineages. Furthermore, as for the cells used for culturing, cells of a single type may be cultured, or cells of two or more types may be co-cultured. Culturing such cells using the microcarrier makes it possible to manufacture medical manufactures and foods expected to have therapeutic effects.

Dissolution

To separate and collect cells from the microcarrier after culture, the microcarrier is dissolved such that cells are easily separated and collected. A microcarrier manufactured by the method of the present disclosure is reacted with a chelating agent. Thus, divalent or higher-valent cation crosslinking alginic acid is removed from the alginic acid, allowing the microcarrier to be dissolved.

EDTA is preferably used as a chelating agent for dissolving the microcarrier. In addition, by adding an alginic acid degrading enzyme and a proteolytic enzyme, in addition to a chelating agent, alginic acid and a gelatin can be hydrolyzed, allowing dissolution more quickly. Meanwhile, from the viewpoint of reducing damage to cells, it is preferable not to use a proteolytic enzyme. It is preferable not to use an alginic acid degrading enzyme to minimize the amount of added substances from the viewpoint of safety management and cost.

Given the above, it is most preferable that the microcarrier can be dissolved with a chelating agent alone.

<Cell Culture Microcarrier>

According to another aspect of the present disclosure, a microcarrier for cell culture is provided. The "microcarrier for cell culture" used herein refers to both a microcarrier before the "drying" step of the method of the present disclosure and a microcarrier (i.e., a microcarrier for cell culture) after swelling the dry microcarrier of the present disclosure (i.e., a dry microcarrier for cell culture) if necessary.

According to another aspect of the present disclosure, a microcarrier for cell culture comprising a gelatin and alginic acid crosslinked with a divalent or higher-valent cation and containing the gelatin inside thereof is provided.

In addition, according to another preferred aspect of the present disclosure, a dry microcarrier for cell culture comprising a gelatin and alginic acid crosslinked with a divalent or higher-valent cation and containing the gelatin inside thereof is provided. It is preferable that the gelatin inside the microcarrier is distributed throughout the microcarrier. Here, for the dry microcarrier, the moisture content in the microcarrier measured by a normal-pressure heat drying method is preferably 50% by mass or less, more preferably 30% by mass or less, still more preferably 25% by mass or less, even more preferably 20% by mass or less, yet more preferably 15% by mass or less, yet more preferably 10% by mass or less, yet more preferably 5% by mass or less, yet more preferably 2% by mass or less, yet more preferably 1% by mass or less. Measurement of the moisture content in the microcarrier by a normal-pressure heat drying method is carried out according to the method described in the Examples.

A gelatin, a divalent or higher-valent cation, alginic acid, and the like contained in the microcarrier for cell culture may be the same as a gelatin, a divalent or higher-valent cation, alginic acid, and the like used in the method of manufacturing a dry microcarrier for cell culture of the present disclosure.

EXAMPLES

Hereinafter, the present disclosure will be described in more detail with reference to Examples; however, the present disclosure is not limited to the following Examples.

Test Example 1: Dry Microcarrier Preparation and Swelling, Cell Culture, and Dissolution

1-1-1

<Preparation of Dry Microcarriers>

A 1% by mass aqueous solution of sodium alginate (194-13321 manufactured by FUJIFILM Corporation) and a 1% by mass aqueous solution of calcium chloride (038-24985 manufactured by FUJIFILM Corporation) were separately prepared.

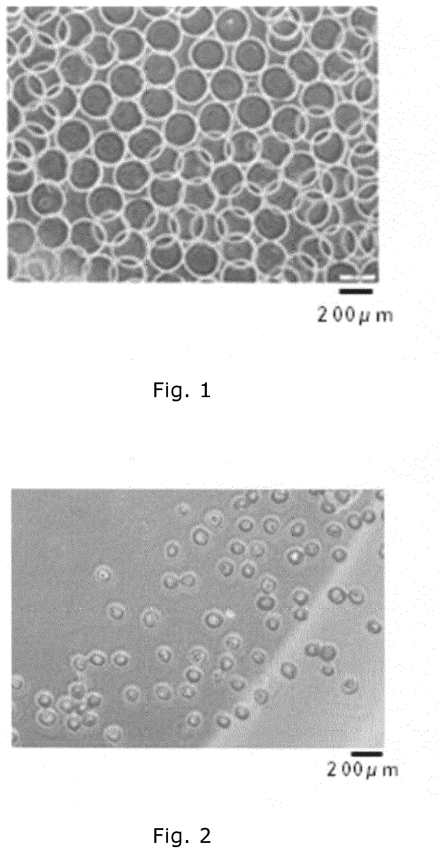

The sodium alginate aqueous solution was added dropwise to the calcium chloride aqueous solution through a 32G syringe needle, thereby preparing gel microparticles of calcium alginate (alginic acid was crosslinked with a calcium ion) having a diameter of about 200 $\mu$m (measured by optical microscopy) (see FIG. 1). The gel microparticles were washed with 70% ethanol and water, and collected using a cell strainer (352340 manufactured by Falcon). The gel microparticles were immersed in a 4% by mass aqueous solution of autoclaved alkali-treated gelatin (G9391-100G manufactured by Sigma-Aldrich) and left at 20°C for 2 hours or more, thereby allowing gelatin to infiltrate the gel microparticles.

Here, the size of the gel microparticle remained about 200 $\mu$m in diameter, and no change in shape was observed. After collecting the gel microparticles with a cell strainer, the gel microparticles were immersed in ethanol and collected with a cell strainer, thereby removing gelatin outside the gel microparticles and encapsulating the gelatin inside the gel microparticles. Here, an optical microscope observation showed that the gel microparticles of calcium alginate were colorless before gelatin was encapsulated therein, and the gelatin aqueous solution was slightly yellowish, while the inside of the gel microparticles after being surrounded with ethanol to encapsulate the gelatin was slightly yellowish, and the outside of the gel microparticles was colorless. It could be confirmed that gelatin was encapsulated inside the gel microparticles.

After the gel microparticles were vacuum-dried in that state, water was removed by thermal drying at 150°C for 2 hours, resulting in a reduced size of about 90 $\mu$m (measured by optical microscopic observation). Thus, powdery dry microcarriers (i.e., dry microcarriers manufactured by the manufacturing method of the present disclosure) were successfully obtained (see FIG. 2). Immediately after vacuum drying, the microcarriers were in the form of a white powder but then turned brown after thermal drying.

1-1-2

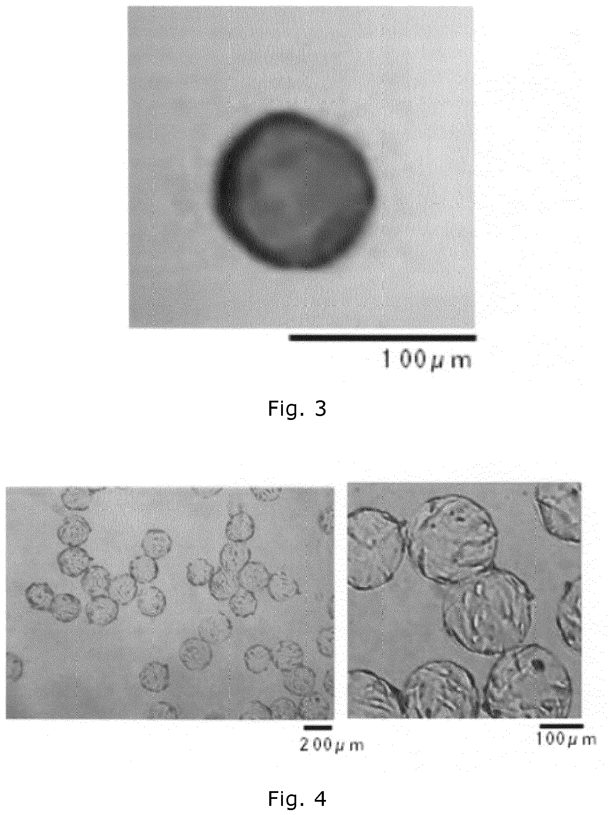

In addition, dry microcarriers were separately prepared as described above, except that fluorescent dyed gelatin (manufactured by Thermo Fisher, G13187) was added at a concentration of 0.01% by mass to the 4% by mass aqueous solution of autoclaved alkali-treated gelatin (G9391-100G manufactured by Sigma-Aldrich) in the above-described method for preparing powdery dry microcarriers. When the prepared dry microcarriers were observed using a Confocal Laser Scanning Microscopy, it was confirmed that the entire inside of the microcarriers was stained and that gelatin was uniformly distributed throughout the dry microcarriers (see FIG. 3: the pinhole was adjusted such that the width of the slice image (cross-sectional view of a dry microcarrier) was sufficiently smaller than the radius of the dried microcarrier). Further, it was observed that the entire inside of the microcarriers was stained similarly in the microcarriers after swelling in <Swelling, Cell Culture, and Dissolution> described below, confirming that gelatin was uniformly distributed throughout the dry microcarriers.

<Swelling, Cell Culture, and Dissolution>

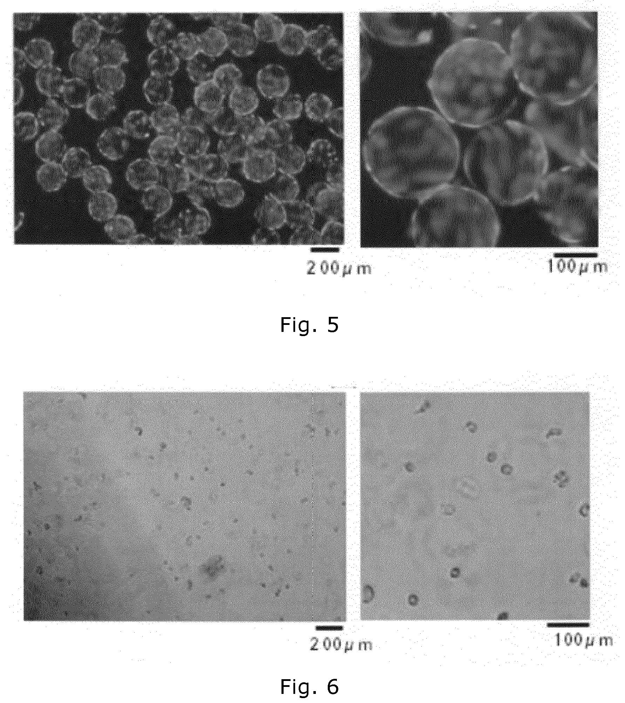

A 10% by mass calcium chloride aqueous solution was added to the MSCGM Bullet Kit (registered trademark) (PT-3001, Lonza) medium in an amount ratio of 1/100. The dry microcarriers prepared above were immersed in the medium and left at 20°C for 30 minutes. Accordingly, the dry microcarriers swelled, resulting in a size of about 200 $\mu$m in diameter, which was substantially the same as the size of gel microparticle of calcium alginate before drying. Subsequently, human mesenchymal stem cells (PT-2501; manufacturer: Lonza) were seeded. As a result, it was confirmed that the cells adhered to and developed on the surfaces of the gel microparticles, and after culturing for one week, the cells proliferated until the cells almost covered the surfaces of the microparticles (see FIGS. 4 and 5). It was confirmed with a microscope that after culturing for one week, the medium was removed, and a 10 mM EDTA solution was added to the gel microparticles, which were then left for about 1 minute, followed by pipetting; thus, the gel microparticles were dissolved such that the cells were separated from the gel microparticles (see FIG. 6).

1-2
Dry microcarriers (obtained after thermal drying) were separately prepared as in 1-1-1 above. A normal-pressure heat drying method (measurement conditions: dryer temperature: 150°C; drying time: 2 hours) was performed on two types of manufactures: manufactures immediately after preparation and stored manufactures (storage conditions: humidity: 100%; 25°C; 3 days). The moisture content of the manufactures immediately after preparation was 0% by mass, and the moisture content of the stored manufactures was 22.5% by mass.

Further, manufactures immediately after preparation and a stored manufactures were separately prepared as described above. The test described in <Swelling, Cell Culture, and Dissolution> above was performed on both manufactures. As a result, results similar to those in the above-described test were observed, confirming that similar effects can be obtained in a moisture content range of from 0% to 22.5% by mass.

1-3
Microcarriers before thermal drying (obtained by immersing gel microparticles in ethanol and collecting them with a cell strainer, thereby removing gelatin outside the gel microparticles and encapsulating the gelatin inside the gel microparticles) were separately prepared in the manner as in 1-1-1 above. The microcarriers were immersed in water. The size of the microcarrier in that state was measured as the "size before drying (average particle size)," which was about 200 $\mu$m in diameter. In addition, dry microcarriers after thermal drying (obtained after performing thermal drying) were separately prepared in the manner as in 1-1-1 above. The size of dry microcarrier after swelling in water at 20°C for 30 minutes was determined to be "size after swelling (average particle size)" for measurement. Here, the size after swelling was about 200 $\mu$m in diameter, which was about the same size as the size after swelling using the medium described in <Swelling, Cell Culture, and Dissolution> in Test Example 1.

The method for measuring the average particle size is as described below.

(Method for Measuring Average Particle Size)

Dry microcarriers were placed in a transparent container with a flat bottom as an observation container, and the dry microcarriers were allowed to precipitate and observed with an optical microscope. The average value of the equivalent volume diameters of all the observed dry microcarriers was determined to be "average particle size." Here, when the dry microcarriers were introduced into the observation container, the bottom area of the container and the amount of dry microcarriers introduced were adjusted for measurement such that 10 or more dry microcarriers could be observed without overlapping.

Further, the difference (%) between the size after swelling and the size before drying in the dry microcarriers was calculated to be 0% based on the following formula.

[Formula 2]

(Difference between size after swelling and size before drying)

(%) = | (Size before drying − Size after swelling) / (Size before drying) × 100 |

Test Example 2: Dry Microcarrier Preparation at Different Gelatin Concentrations and Culture Evaluation

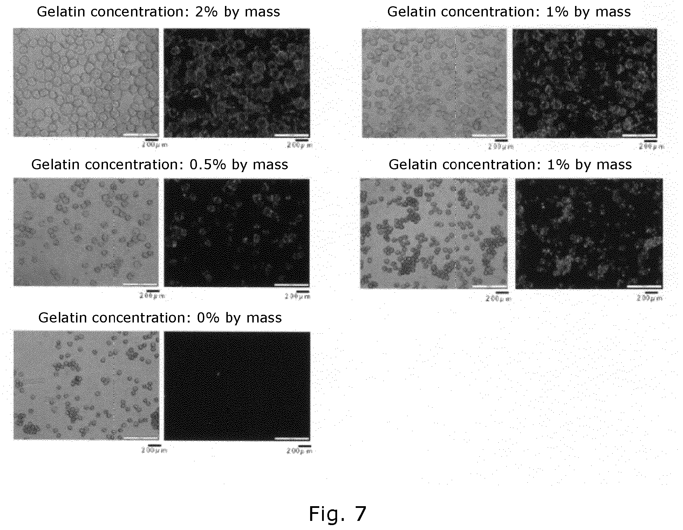

Dry microcarriers were prepared, followed by swelling, culture, and dissolution as described in 1-1-1 of Test Example 1, except that the gelatin concentration of a gelatin aqueous solution for immersing gel microparticles of calcium alginate was set to 0% by mass, 0.1% by mass, 0.5% by mass, 1% by mass, or 2% by mass (see FIG. 7).

For dry microcarriers, which were prepared as in Test Example 1 except that the gelatin concentration was set to 0% by mass, 0.1% by mass, 0.5% by mass, 1% by mass, or 2% by mass, the difference between the size of microcarrier after swelling (size after swelling) and the size of gel microparticle of alginic acid immersed in water before drying (size before drying) was calculated as in 1-3 above.

The results were as shown in Table 1 below. Here, the size after swelling was about the same size as the size after swelling using the medium described in <Swelling, Cell Culture, and Dissolution> in Test Example 1.

[Table 1]

| Gelatin concentration (% by mass) | Size before drying (average particle size) | Size after swelling (average particle size) | Difference between size after swelling and size before drying (%) |
|---|---|---|---|
| 0 | 200 | 60 | 70 |
| 0.1 | 200 | 70 | 65 |
| 0.5 | 200 | 120 | 40 |
| 1 | 200 | 150 | 25 |
| 2 | 200 | 200 | 0 |

In a case in which the gelatin concentration of a gelatin aqueous solution for immersing gel microparticles of calcium alginate was set to 0% by mass, 0.1% by mass, 0.5% by mass, 1% by mass, and 2% by mass as listed in Table 1, the microparticle size of dry microcarrier after swelling (measured by optical microscopic observation) became closer to the size before drying as the gelatin concentration increased and was about 60 $\mu$m, about 70 $\mu$m, about 120 $\mu$m, about 150 $\mu$m, and about 200 $\mu$m, respectively. It can be seen that as gelatin is contained in the microparticles, favorable swelling is facilitated.

Here, in the cases in which the gelatin concentration of a gelatin aqueous solution for immersing gel microparticles of calcium alginate was 0% by mass and 0.1% by mass, agglomeration of microparticles was observed after swelling of dry microcarriers, while substantially no agglomeration was observed in the other cases.

Regarding cell adhesiveness, in the case in which the gelatin concentration of a gelatin aqueous solution for immersing gel microparticles of calcium alginate was 0% by mass, substantially no cell adhesion was observed.

Regarding cell adhesiveness, in the cases in which the gelatin concentration of a gelatin aqueous solution for immersing gel microparticles of calcium alginate was 0.1% by mass and 0.5% by mass, cells did not proliferate to the extent that they covered the surfaces of the microparticles, although cell adhesion was observed.

Regarding cell adhesiveness, in the cases in which the gelatin concentration of a gelatin aqueous solution for immersing gel microparticles of calcium alginate was 1% by mass and 2% by mass, cell adhesion was observed, and cells were confirmed to proliferate to the extent that they covered the surfaces of the microparticles.

The results above revealed that in the case in which the gelatin concentration of a gelatin aqueous solution for immersing gel microparticles of calcium alginate was 0.5% by mass or more, the swelling was observed to the extent that the size of dry microcarrier after swelling (average particle size) was with a difference of 50% or less of the size of dry microcarrier before drying (average particle size), and the swelling was favorable with substantially no agglomeration.

In addition, in the case in which the gelatin concentration of a gelatin aqueous solution for immersing gel microparticles of calcium alginate was 0.1% by mass or more, it was found that the gelatin concentration of a gelatin aqueous solution for immersing gel microparticles of calcium alginate significantly improved, compared with the case of 0% by mass, also regarding cell adhesiveness and proliferative capacity.

Further, in the case in which the gelatin concentration of a gelatin aqueous solution for immersing gel microparticles of calcium alginate was 1% by mass or more, it was found that swelling was favorable, and also regarding cell adhesiveness and proliferative capacity, favorable proliferation occurred to the extent that the surfaces of the swollen microcarriers were almost entirely covered.

It was also confirmed with a microscope that after culturing for one week, the medium was removed, and a 10 mM EDTA solution was added to the gel microparticles, which were then left for about 1 minute, followed by pipetting; thus, the gel microparticles were dissolved such that the cells were separated from the gel microparticles, when the gelatin concentration was any of 0% by mass, 0.1% by mass, 0.5% by mass, 1% by mass, and 2% by mass.

As in Test Example 1, evaluation was made at a gelatin concentration of 4% by mass. It was confirmed that the size of dry microcarrier after swelling was about 200 μm, the swelling was favorable, cell adhesion was observed, and cells proliferated to the extent that they covered the surfaces of microparticles. Further, it was confirmed with a microscope that after culturing for one week, the medium was removed, and a 10 mM EDTA solution was added to the gel microparticles, which were then left for about 1 minute, followed by pipetting; thus, the gel microparticles were dissolved such that the cells were separated from the gel microparticles under all conditions for gelatin concentration examined herein.

Test Example 3: Examination of Amount of Gelatin in Dry Microcarriers at Different Gelatin Concentrations

Dry microcarriers (dry microcarriers manufactured by the method of the present disclosure) were prepared in the same manner as in 1-1-1 of Test Example 1 and Test Example 2, and elemental analysis thereof was carried out by the carbon, hydrogen and nitrogen elemental analysis or the reduced pressure chemiluminescence method, thereby measuring the amount of gelatin inside the dry microcarriers (% by mass). The specific procedures and measurement conditions for the carbon, hydrogen and nitrogen elemental analysis and the reduced pressure chemiluminescence method were as follows.

(Carbon, Hydrogen and Nitrogen Elemental Analysis Method)

The sample was accurately weighed to the nearest 0.0001 mg, and simultaneous carbon, hydrogen and nitrogen analysis was performed.

<Measurement Conditions>

System: vario MICRO cube (manufactured by Elementar)
Combustion furnace: 1150°C
Reduction furnace: 850°C
Helium flow rate: 200 mL/min
Oxygen flow rate: from 25 to 30 mL/min

(Reduced Pressure Chemiluminescence Method)

A sample was weighed and introduced into an analyzer, and nitrogen monoxide manufactured by thermal decomposition and oxidation was measured using a chemiluminescence method. For quantitative determination, the concentration was automatically calculated in advance using a calibration curve prepared using a pyridine standard solution.

<Measurement Conditions>

Apparatus: Trace nitrogen analyzer ND-100 (manufactured by Mitsubishi Chemical Corporation)

Electric furnace temperature (horizontal reactor)

Pyrolysis part: 800°C
Catalyst part: 900°C

$O_2$ flow rate (main/sub): 300 mL/min (setting)/300 mL/min (setting)
Ar flow rate: 400 mL/min (setting)
Sens.: Low

Here, for dry microcarriers prepared with gelatin alone and dry microcarriers prepared in a case in which the gelatin concentration of a gelatin aqueous solution for immersing gel microparticles of calcium alginate was 4% by mass, 2% by mass, 1% by mass, or 0.5% by mass, the nitrogen content (% by mass) in dry microcarriers was measured by carbon, hydrogen and nitrogen elemental analysis. In addition, for dry microcarriers prepared in a case in which the gelatin concentration of a gelatin aqueous solution for immersing gel microparticles of calcium alginate was 0.1% by mass or 0% by mass (alginic acid alone), the nitrogen content (% by mass) in dry microcarriers was measured by the reduced pressure chemiluminescence method. The amount of gelatin (% by mass) was calculated from the nitrogen contents (% by mass) in the cases of alginic acid alone and gelatin alone. Specifically, in the carbon, hydrogen and nitrogen elemental analysis or the reduced pressure chemiluminescence method, the nitrogen content (Y) of dry microcarriers was measured based on the nitrogen composition ratio as the nitrogen content (% by mass), and the amount of gelatin (A) was calculated with the gelatin composition ratio of dry microcarriers as the gelatin content (% by mass) based on the nitrogen content (Z) in the case of alginic acid alone and the nitrogen content (X) in the case of gelatin alone according to the following formula.

$$A = (Y-Z)/(X-Z)$$

The nitrogen content and amount of gelatin in dry microcarriers and the results of swelling and culture above are shown in Table 2 for each gelatin concentration condition. In the case of gelatin alone, the nitrogen content in dry microcarriers was 16.2% by mass. In the case in which the gelatin concentration of a gelatin aqueous solution for immersing gel microparticles of calcium alginate was 0.5% by mass, the nitrogen content and the amount of gelatin in dry microcarriers were 3.9% by mass and 24% by mass, respectively. The results in Table 2 revealed that as the gelatin concentration of a gelatin aqueous solution for immersing gel microparticles of calcium alginate increases, the nitrogen content and the amount of gelatin in dry microcarriers also increase. Evaluation criteria for "swelling" and "cell adhesiveness and proliferative capacity" used herein are as follows. Methods for evaluating "swelling" and "cell adhesiveness and proliferative capacity" were implemented according to the methods described in Test Example 2.

(1) Swelling

<Evaluation Criteria>

A: Swelling takes place to the extent that the size of dry microcarrier after swelling (average particle size) is with a difference of 50% or less of the size of dry microcarrier before drying (average particle size).
B: Swelling takes place to the extent that the size of dry microcarrier after swelling (average particle size) is with a difference of more than 50% and less than 70% of the size of dry microcarrier before drying (average particle size).
C: Swelling takes place to the extent that the size of dry microcarrier after swelling (average particle size) is with a difference of 70% or more of the size of dry microcarrier before drying (average particle size).

(2) Cell Adhesiveness and Proliferative Capacity

<Evaluation Criteria>

A: Cell adhesion is observed, and cells are confirmed to proliferate to the extent that they cover the microparticle surface.
B: Cell adhesion is observed, and cells proliferate although not to the extent that they cover the microparticle surface.
C: Almost no cell adhesion is observed.

<Amounts of Gelatin in Dry Microcarriers at Different Gelatin Concentrations>

Dry microcarriers were prepared by the same method described in Test Example 2 and subjected to XPS analysis. Thus, the amount of gelatin on the surfaces of the dry microcarriers was measured as the value of N 1s/C 1s (atomic %/atomic %). Here, N 1s is a value derived from nitrogen atoms from gelatin, and C 1s is a value derived from carbon atoms of the entire dry microcarriers. Al Ka radiation was used as a photoelectron excitation source for XPS analysis, and ESCA-3400 (manufactured by Kratos Analytical Ltd) was used as a specific apparatus.

Table 3 shows the amounts of gelatin on the surfaces of dry microcarriers prepared at different gelatin concentrations in a gelatin aqueous solution, in which gel microparticles of calcium alginate were immersed, with the value of N 1s/C 1s (atomic %/atomic %) and the results of nitrogen contents, swelling, and culture shown in Table 2.

In addition, Table 2 lists the value of $\beta/\alpha$ where N 1s/C 1s (atomic %/atomic %) obtained by XPS analysis is designated as $\alpha$ and the nitrogen content (% by mass) obtained by the carbon, hydrogen and nitrogen elemental analysis or the reduced pressure chemiluminescence method is designated as $\beta$. For dry microcarriers prepared with gelatin alone and dry microcarriers prepared by setting the gelatin concentration of a gelatin aqueous solution for immersing gel microparticles of calcium alginate to 4% by mass, 2% by mass, 1% by mass, and 0.5% by mass, the nitrogen content $\beta$ (% by mass) was measured by carbon, hydrogen and nitrogen elemental analysis. In addition, for dry microcarriers prepared by setting the gelatin concentration of a gelatin aqueous solution for immersing gel microparticles of calcium alginate to 0.1% by mass and 0% by mass (alginic acid alone), the nitrogen content $\beta$ (% by mass) was measured by the reduced pressure chemiluminescence method. Each of the above-described values of $\alpha$ and $\beta$ was measured when the dry microcarriers were in an unpulverized state (in the state before pulverization).

Here, considering the characteristics (measurement thresholds) of the carbon, hydrogen and nitrogen elemental analysis and the reduced pressure chemiluminescence method, dry microcarriers in the form of having a nitrogen content of 0.5% by mass or less were measured by the reduced pressure chemiluminescence method and dry microcarriers in the form of having a nitrogen content of more than 0.5% by mass were measured by the carbon, hydrogen and nitrogen elemental analysis. In addition, the specific procedures and measurement conditions for the carbon, hydrogen and nitrogen elemental analysis and the reduced pressure chemiluminescence method were the same as the procedures and measurement conditions for the carbon, hydrogen and nitrogen elemental analysis and the reduced pressure chemiluminescence method in this Test Example described above, respectively.

The results of $\alpha$ in Table 3 show that there is substantially no increase in the amount of gelatin on the surfaces of dry microcarriers in a case in which the gelatin concentration of a gelatin aqueous solution for immersing gel microparticles of calcium alginate is 0.5% by mass or more.

Meanwhile, the results of $\beta$ in Table 3 show that the nitrogen content ($\beta$) (the amount of gelatin) in the entire dry microcarriers increases by increasing the gelatin concentration of a gelatin aqueous solution for immersing gel microparticles of calcium alginate, revealing that gelatin is carried inside dry microcarriers depending on the gelatin concentration.

It was also revealed that, as described above, there is substantially no increase in $\alpha$ (the element content of nitrogen on the dry microcarrier surface) in a case in which the gelatin concentration of a gelatin aqueous solution for immersing gel microparticles of calcium alginate is 0.5% by mass or more; thus, as long as $\beta/\alpha$ is 4.96 or more, gelatin is carried inside dry microcarriers. Further, dry microcarriers prepared by the same method described in Test Example 2 above were ground. XPS analysis was performed before and after pulverization, thereby determining $\alpha$ (N 1s/C 1s) before and after pulverization. Al K$\alpha$ radiation was used as a photoelectron excitation source for XPS analysis, and ESCA-3400 (manufactured by Kratos Analytical Ltd) was used as a specific apparatus. Table 3 below shows the values of $\alpha$ (after pulverization)/a (before pulverization) where N 1s/C 1s (atomic %/atomic %) before pulverization is designated as $\alpha$ (before pulverization) and N 1s/C 1s (atomic %/atomic %) after pulverization is designated as $\alpha$ (after pulverization). It was found that gelatin is carried inside dry microcarriers at an $\alpha$ (after pulverization)/a (before pulverization) value of 0.08 or more.

[Table 2]

| Gelatin concentration (% by mass) | Swelling | Cell adhesiveness and proliferative capacity | Nitrogen content ($\beta$) (% by mass) | Amount of gelatin (% by mass) |
|---|---|---|---|---|
| 0 | C | C | 0.031 | 0 |
| 0.1 | B | B | 0.43 | 2.5 |
| 0.5 | A | B | 3.9 | 24 |
| 1 | A | A | 6.7 | 41 |
| 2 | A | A | 9.4 | 58 |
| 4 | A | A | 11.5 | 71 |

[Table 3]

| Gelatin concentrat ion (% by mass) | Swe lling | Cell adhesiven ess and proliferati ve capacity | Nitrogen content ($\beta$) (% by mass) | N1s/C1s($\alpha$) (atomi c%/ atom ic%) | $\beta/\alpha$ | $\alpha$ (after pulveriza tion)/$\alpha$ (before pulveriza tion) |
|---|---|---|---|---|---|---|
| 0 | C | C | 0.031 | 0.03 | 1.035 | 0.02 |
| 0.1 | B | B | 0.43 | 0.087 | 4.965 | 0.08 |
| 0.5 | A | B | 3.9 | 0.144 | 27.04 | 0.43 |
| 1 | A | A | 6.7 | 0.149 | 44.98 | 0.69 |
| 2 | A | A | 9.4 | 0.153 | 61.24 | 0.91 |

Test Example 4: Storage Stability Test

The storage stability test was performed on dry microcarriers prepared at a gelatin concentration of 4% by mass by the same method described in 1-1-1 of Test Example 1. Specifically, after storing the prepared dry microcarriers for 18 months at room temperature, swelling, cell culture, and dissolution of the dry microcarriers were performed by the methods described in 1-1 of Test Example 1. As a result, regarding swelling, cell culture, and dissolution abilities, dry microcarriers stored at room temperature for 18 months were confirmed to exert the abilities comparable to those of the dry microcarriers before storage. The results showed that the dry microcarrier of the present disclosure has favorable storage stability.

Test Example 5: Microcarrier Preparation and Swelling, Cell Culture, and Dissolution of Microcarriers

Microcarriers were prepared by the same method in Test Example 1, except that the infiltration of gelatin into gel microparticles was carried out by immersing gel microparticles in a 2% by mass gelatin aqueous solution for one day, and the drying step was omitted during dry microcarrier preparation in Test Example 1. Mouse skeletal myoblasts were seeded on the prepared microcarriers and cultured in a DMEM medium. As a result, cell adhesion and proliferation on the microcarriers were confirmed.

Microcarriers were prepared by the same method described above, except that the 1% by mass aqueous solution of calcium chloride was changed to a 1% by mass aqueous solution of magnesium chloride, a 1% by mass aqueous solution of iron(II) chloride, and a 1% by mass aqueous solution of iron(III) chloride during the above-described microcarrier preparation. Mouse skeletal myoblasts were seeded on the prepared microcarriers and cultured in a DMEM medium. As a result, cell adhesion and proliferation on the microcarriers were confirmed.

Cell adhesiveness and proliferative capacity of the above-described microcarriers were evaluated by the same method described in Test Example 3. All results corresponded to "B: Cells do not proliferate to the extent that they cover the surfaces of the microparticles, although cell adhesion is observed."

Although the drying step was omitted during the preparation of microcarriers in this Test Example, it is considered that cell adhesiveness and storage stability can improve with the drying step. In other words, drying microcarriers allows the volume of each microparticle of alginic acid to decrease such that the microparticle is condensed and alginic acid constituting the microparticle of alginic acid is brought into close contact with a cell adhesive substance such as gelatin. It is thus thought that a physical crosslink is formed between the alginic acid and the cell adhesion substance, and the two are firmly bonded to each other, thereby improving cell adhesion. Further, by drying the microcarrier, the microcarrier becomes substantially free of liquid components such as water. It is thus thought that a chemical reaction such as hydrolysis caused by liquid components and the proliferation of microorganisms such as bacteria are suppressed, thereby improving storage stability. Therefore, considering that favorable cell adhesiveness and high storage stability were achieved in microcarriers for which the drying step was omitted, it is thought that in a case in which the drying step is performed on the microcarriers, there is a high probability that favorable cell adhesiveness and high storage stability will be achieved at the same or greater level of the case in which the drying step is omitted.

Another aspect of the present disclosure relates to the following [1] to [18].

[1] A method of manufacturing a dry microcarrier for cell culture, comprising:

immersing a gel microparticle of alginic acid containing a divalent or higher-valent cation in a solvent containing a gelatin; and

forming a dry microcarrier by drying the gel microparticle of alginic acid immersed in the solvent,
wherein the dry microcarrier for cell culture comprises a gelatin and alginic acid crosslinked with a divalent or higher-valent cation, and contains the gelatin inside thereof.

[2] The method according to [1], which comprises immersing the gel microparticle of alginic acid immersed in the solvent containing a gelatin in a poor solvent for gelatin before drying.

[3] The method according to [2], wherein the poor solvent is a volatile solvent.

[4] The method according to any one of [1] to [3], wherein the dry microcarrier for cell culture consists of a gelatin and a salt consisting of alginic acid crosslinked with a divalent or higher-valent cation.

[5] The method according to any one of [1] to [4], wherein a content of the gelatin in the solvent is more than 0.1% by mass with respect to a total amount of the solvent.

[6] The method according to any one of [1] to [5], wherein a content of the gelatin in the entire dry microcarrier for cell culture is 2.5% by mass or more with respect to a total amount of the dry microcarrier for cell culture.

[7] The method according to any one of [1] to [6], wherein a nitrogen content in the dry microcarrier for cell culture is 0.43% by mass or more with respect to a total amount of the dry microcarrier for cell culture.

[8] The method according to any one of [1] to [7], wherein the divalent or higher-valent cation is at least one selected from the group consisting of a calcium ion, a magnesium ion, a divalent iron ion, a trivalent iron ion, a divalent silver ion, a trivalent silver ion, a copper ion, a zinc ion, a selenium ion, and other trace element ions essential for humans.

[9] The method according to any one of [1] to [8], wherein the gelatin contained inside the dry microcarrier for cell culture is bonded to alginic acid.

[10] A dry microcarrier for cell culture, which is manufactured by the method according to any one of [1] to [9].

[11] A dry microcarrier for cell culture, which comprises a gelatin and alginic acid crosslinked with a divalent or higher-valent cation and contains the gelatin inside thereof.

[12] A dry microcarrier for cell culture, which comprises a gelatin and alginic acid crosslinked with a divalent or higher-valent cation and contains the gelatin inside thereof,
wherein the dry microcarrier for cell culture has an average particle size of 1 mm or less.

[13] The dry microcarrier for cell culture according to [11] or [12], wherein the gelatin is distributed throughout the inside of the dry microcarrier for cell culture.

[14] A dry microcarrier for cell culture, which comprises a gelatin and alginic acid crosslinked with a divalent or higher-valent cation,

wherein when N 1s/C 1s (atomic %/atomic %) obtained by X-ray photoelectron spectroscopy (XPS) for the dry microcarrier for cell culture is designated as $\alpha$, and
a nitrogen content (% by mass) obtained by carbon, hydrogen and nitrogen elemental analysis method or a reduced pressure chemiluminescence method for the dry microcarrier for cell culture is designated as $\beta$,
$\beta$ (before pulverization)/a (before pulverization) is 4.96 or more, or
$\alpha$ (after pulverization)/o (before pulverization) is 0.08 or more (wherein "$\alpha$ (before pulverization)" and "$\beta$ (before pulverization)" represent N 1s/C 1s (atomic %/atomic %) and nitrogen content (% by mass) for the dry microcarrier for cell culture, respectively, and "$\alpha$ (after pulverization)" represents N 1s/C 1s (atomic %/atomic %) for a powder obtained by grinding the dry microcarrier for cell culture).

[15] The dry microcarrier for cell culture according to [14], wherein the $\beta$ (before pulverization)/o (before pulverization) is 4.96 or more, and
the $\alpha$ (after pulverization)/o (before pulverization) is 0.08 or more.

[16] A dry microcarrier for cell culture, which comprises a gelatin and alginic acid crosslinked with a divalent or higher-valent cation,

wherein when N 1s/C 1s (atomic %/atomic %) obtained by X-ray photoelectron spectroscopy (XPS) for the dry microcarrier for cell culture is designated as $\alpha$, and
a nitrogen content (% by mass) obtained by carbon, hydrogen and nitrogen elemental analysis method or a reduced pressure chemiluminescence method for the dry microcarrier for cell culture is designated as $\beta$,
$\beta$ (before pulverization)/a (before pulverization) is 4.96 or more, and
$\alpha$ (after pulverization)/o (before pulverization) is 0.08 or more (wherein "$\alpha$ (before pulverization)" and "$\beta$ (before pulverization)" represent N 1s/C 1s (atomic %/atomic %) and nitrogen content (% by mass) for the dry microcarrier for cell culture, respectively, and "$\alpha$ (after pulverization)" represents N 1s/C 1s (atomic %/atomic %) for a powder obtained by grinding the dry microcarrier for cell culture).
wherein the dry microcarrier for cell culture has an average particle size of 1 mm or less.

[17] The dry microcarrier for cell culture according to any one of [10] to [16], wherein a content of the gelatin in the entire dry microcarrier for cell culture is 2.5% by mass or more with respect to a total amount of the dry microcarrier for cell culture.

[18] The dry microcarrier for cell culture according to any one of [10] to [17], wherein a nitrogen content in the dry microcarrier for cell culture is 0.43% by mass or more with respect to a total amount of the dry microcarrier for cell culture.

**Claims**

1. A method of manufacturing a dry microcarrier for cell culture, comprising:

   immersing a gel microparticle of alginic acid containing a divalent or higher-valent cation in a solvent containing a gelatin; and
   forming a dry microcarrier by drying the gel microparticle of alginic acid immersed in the solvent,
   wherein the dry microcarrier for cell culture comprises a gelatin and alginic acid crosslinked with a divalent or higher-valent cation, and contains the gelatin inside thereof.

2. The method according to claim 1, which comprises immersing the gel microparticle of alginic acid immersed in the solvent containing a gelatin in a poor solvent for gelatin before drying.

3. The method according to claim 2, wherein the poor solvent is a volatile solvent.

4. The method according to claim 1, wherein the dry microcarrier for cell culture consists of a gelatin and a salt consisting of alginic acid crosslinked with a divalent or higher-valent cation.

5. The method according to claim 1, wherein a content of the gelatin in the solvent is more than 0.1% by mass with respect to a total amount of the solvent.

6. The method according to claim 1, wherein a content of the gelatin in the entire dry microcarrier for cell culture is 2.5% by mass or more with respect to a total amount of the dry microcarrier for cell culture.

7. The method according to claim 1, wherein a nitrogen content in the dry microcarrier for cell culture is 0.43% by mass or more with respect to a total amount of the dry microcarrier for cell culture.

8. The method according to claim 1, wherein the divalent or higher-valent cation is at least one selected from the group consisting of a calcium ion, a magnesium ion, a divalent iron ion, a trivalent iron ion, a divalent silver ion, a trivalent silver ion, a copper ion, a zinc ion, a selenium ion, and other trace element ions essential for humans.

9. The method according to claim 1, wherein the gelatin contained inside the dry microcarrier for cell culture is bonded to alginic acid.

10. A dry microcarrier for cell culture, which is manufactured by the method according to claim 1.

11. A dry microcarrier for cell culture, which comprises a gelatin and alginic acid crosslinked with a divalent or higher-valent cation and contains the gelatin inside thereof.

12. A dry microcarrier for cell culture, which comprises a gelatin and alginic acid crosslinked with a divalent or higher-valent cation and contains the gelatin inside thereof,
    wherein the dry microcarrier for cell culture has an average particle size of 1 mm or less.

13. The dry microcarrier for cell culture according to claim 11, wherein the gelatin is distributed throughout the inside of the dry microcarrier for cell culture.

14. A dry microcarrier for cell culture, which comprises a gelatin and alginic acid crosslinked with a divalent or higher-valent cation,

    wherein when N 1s/C 1s (atomic %/atomic %) obtained by X-ray photoelectron spectroscopy (XPS) for the dry microcarrier for cell culture is designated as $\alpha$, and

a nitrogen content (% by mass) obtained by carbon, hydrogen and nitrogen elemental analysis method or a reduced pressure chemiluminescence method for the dry microcarrier for cell culture is designated as $\beta$,

$\beta$ (before pulverization)/a (before pulverization) is 4.96 or more, or

$\alpha$ (after pulverization)/o (before pulverization) is 0.08 or more (wherein "$\alpha$ (before pulverization)" and "$\beta$ (before pulverization)" represent N 1s/C 1s (atomic %/atomic %) and nitrogen content (% by mass) for the dry microcarrier for cell culture, respectively, and "$\alpha$ (after pulverization)" represents N 1s/C 1s (atomic %/atomic %) for a powder obtained by grinding the dry microcarrier for cell culture).

15. The dry microcarrier for cell culture according to claim 14, wherein the $\beta$ (before pulverization)/o (before pulverization) is 4.96 or more, and

the $\alpha$ (after pulverization)/o (before pulverization) is 0.08 or more.

16. A dry microcarrier for cell culture, which comprises a gelatin and alginic acid crosslinked with a divalent or higher-valent cation,

wherein when N 1s/C 1s (atomic %/atomic %) obtained by X-ray photoelectron spectroscopy (XPS) for the dry microcarrier for cell culture is designated as $\alpha$, and

a nitrogen content (% by mass) obtained by carbon, hydrogen and nitrogen elemental analysis method or a reduced pressure chemiluminescence method for the dry microcarrier for cell culture is designated as $\beta$,

$\beta$ (before pulverization)/a (before pulverization) is 4.96 or more, and

$\alpha$ (after pulverization)/o (before pulverization) is 0.08 or more (wherein "$\alpha$ (before pulverization)" and "$\beta$ (before pulverization)" represent N 1s/C 1s (atomic %/atomic %) and nitrogen content (% by mass) for the dry microcarrier for cell culture, respectively, and "$\alpha$ (after pulverization)" represents N 1s/C 1s (atomic %/atomic %) for a powder obtained by grinding the dry microcarrier for cell culture), and

wherein the dry microcarrier for cell culture has an average particle size of 1 mm or less.

17. The dry microcarrier for cell culture according to claim 10, wherein a content of the gelatin in the entire dry microcarrier for cell culture is 2.5% by mass or more with respect to a total amount of the dry microcarrier for cell culture.

18. The dry microcarrier for cell culture according to claim 10, wherein a nitrogen content in the dry microcarrier for cell culture is 0.43% by mass or more with respect to a total amount of the dry microcarrier for cell culture.

Fig. 1

Fig. 2

100μm

Fig. 3

200μm    100μm

Fig. 4

Fig. 5

Fig. 6

Gelatin concentration: 2% by mass

Gelatin concentration: 1% by mass

Gelatin concentration: 0.5% by mass

Gelatin concentration: 1% by mass

Gelatin concentration: 0% by mass

Fig. 7

## INTERNATIONAL SEARCH REPORT

| International application No. |
| --- |
| **PCT/JP2022/019966** |

| A. | CLASSIFICATION OF SUBJECT MATTER |
| --- | --- |

*C12N 11/02*(2006.01)i; *C07K 17/14*(2006.01)i; *C12N 5/07*(2010.01)i
FI:   C12N11/02; C07K17/14; C12N5/07

According to International Patent Classification (IPC) or to both national classification and IPC

| B. | FIELDS SEARCHED |
| --- | --- |

Minimum documentation searched (classification system followed by classification symbols)

C12N11/02; C07K17/14; C12N5/07

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2014-502989 A (VESALE PHARMA SA) 06 February 2014 (2014-02-06)<br>example 11 | 1-18 |
| X | JP 3-87172 A (SNOW BRAND MILK PROD CO LTD) 11 April 1991 (1991-04-11)<br>claims, Industrial application field, example 1 | 1-18 |
| A | JP 2018-516598 A (CAVIAROLI, S.L.) 28 June 2018 (2018-06-28)<br>entire text | 1-18 |

☐ Further documents are listed in the continuation of Box C.      ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **09 June 2022** | **21 June 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2022/019966**

| Patent document cited in search report | | | Publication date (day/month/year) | Patent family member(s) | | | Publication date (day/month/year) |
|---|---|---|---|---|---|---|---|
| JP | 2014-502989 | A | 06 February 2014 | US 2014/0010918 A1 example 11 | | | |
| JP | 3-87172 | A | 11 April 1991 | (Family: none) | | | |
| JP | 2018-516598 | A | 28 June 2018 | US 2018/0146704 A1 entire text | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010138702 A
- JP 2018516578 A
- JP H581 A